(19) Europäisches Patentamt
European Patent Office
Office européen des brevets

(11) **EP 4 497 461 A1**

(12) **EUROPEAN PATENT APPLICATION**

(43) Date of publication:
**29.01.2025 Bulletin 2025/05**

(21) Application number: **24190001.8**

(22) Date of filing: **22.07.2024**

(51) International Patent Classification (IPC):
***A61M 16/04*** *(2006.01)*

(52) Cooperative Patent Classification (CPC):
(C-Sets available)
**A61M 16/0461; A61M 16/0475;** A61M 16/0415;
A61M 2210/0618; A61M 2210/065          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.07.2023 TW 112127810**

(71) Applicant: **Chen, Tien-Sheng
Taipei City 112 (TW)**

(72) Inventor: **Chen, Tien-Sheng
Taipei City 112 (TW)**

(74) Representative: **Viering, Jentschura & Partner
mbB
Patent- und Rechtsanwälte
Am Brauhaus 8
01099 Dresden (DE)**

(54) **NASOPHARYNGEAL AIRWAY**

(57)     A nasopharyngeal airway (1) has multiple fenestrations(10, 10a, 10b) and a curved tube(20). The curved tube(20) has a curved front section(22) with a front end(221). Multiple fenestrations(10, 10a, 10b) are provided in the curved front section(22). The curved tube(20) has a curved tube length 1 D1 and the curved front section(22) has a front length D2, where

$$D2 \leq \frac{1}{3}D1 .$$

FIG. 1

EP 4 497 461 A1

(52) Cooperative Patent Classification (CPC): (Cont.)

C-Sets
A61M 2210/0618, A61M 2210/005

**Description**

BACKGROUND OF THE INVENTION

Field of the Invention

The present invention relates to a nasopharyngeal airway; more particularly, the present invention relates to a nasopharyngeal airway having multiple fenestrations provided in a curved front section.

Description of the Related Art

A nasopharyngeal airway can help establish a smooth breathing passage from a patient's nostril to the tongue base or laryngopharynx. When a patient is dangerously ill or unconscious, the patient's tongue base may slide back to obstruct the tracheal airway. At this time, it is necessary to insert a nasopharyngeal airway to secure a smooth breathing passage for the patient. However, the ventilation hole of a conventional nasopharyngeal airway is provided only in the tip of a tube body of the conventional nasopharyngeal airway, and the nasopharyngeal airway has a fixed length. As a result, after the nasopharyngeal airway is inserted into the patient's nasal cavity, the healthcare provider will usually encounter difficulty in causing the ventilation hole provided in the tip of the nasopharyngeal airway to pass the tongue base and enter the patient's laryngopharynx. Improper positioning of the ventilation hole will result in failure to establish a smooth breathing passage for providing sufficient oxygen to the patient. Therefore, there is a need to provide a nasopharyngeal airway to mitigate and/or obviate the aforementioned problems.

SUMMARY OF THE INVENTION

It is an object of the present invention to provide a nasopharyngeal airway having multiple fenestrations provided in a curved front section.

To achieve the abovementioned object, the nasopharyngeal airway of the present invention includes a plurality of fenestrations and a curved tube. The curved tube includes a curved front section, and the curved front section has a front end. The plurality of fenestrations are provided in the curved front section. The curved tube has a curved tube length D1 and the curved front section has a front length D2, where

$$D2 \leq \frac{1}{3}D1$$

.

According to the characteristics of the present invention, by means of providing a plurality of fenestrations in the curved front section of the nasopharyngeal airway, when the nasopharyngeal airway is placed into a human pharynx and the front end of the nasopharyngeal airway enters a human esophagus, even if the healthcare provider cannot precisely place the ventilation holes of the nasopharyngeal airway of the present invention into the patient's upper airway, the plurality of fenestrations provided in the curved front section can still provide oxygen to the patient, thereby resolving the problem that the conventional nasopharyngeal airway cannot be precisely placed in an oxygen provision position without difficult manipulations.

BRIEF DESCRIPTION OF THE DRAWINGS

FIG. 1 illustrates a perspective view of a nasopharyngeal airway according to one embodiment of the present invention;
FIG. 2 illustrates another perspective view of the nasopharyngeal airway according to one embodiment of the present invention;
FIG. 3 illustrates a bottom view of the nasopharyngeal airway according to one embodiment of the present invention;
FIG. 4 illustrates a cross-sectional drawing of an curved front section of the nasopharyngeal airway according to the present invention; and
FIG. 5 illustrates a cross-sectional schematic drawing of the nasopharyngeal airway being placed into a human pharynx.

DETAILED DESCRIPTION OF THE EMBODIMENT

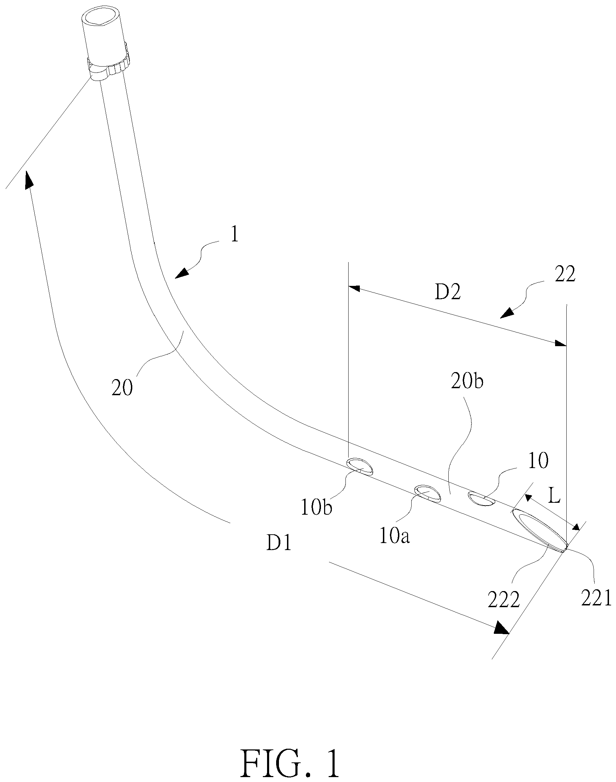

In order to better understand the technical content of the present invention, preferred embodiments are described as follows. Please refer to FIG. 1 to FIG. 5, which illustrate perspective views, a bottom view and a cross-sectional drawing of a curved front section of a nasopharyngeal airway according to the present invention, as well as a cross-sectional schematic drawing of the nasopharyngeal airway inserted into a human pharynx.

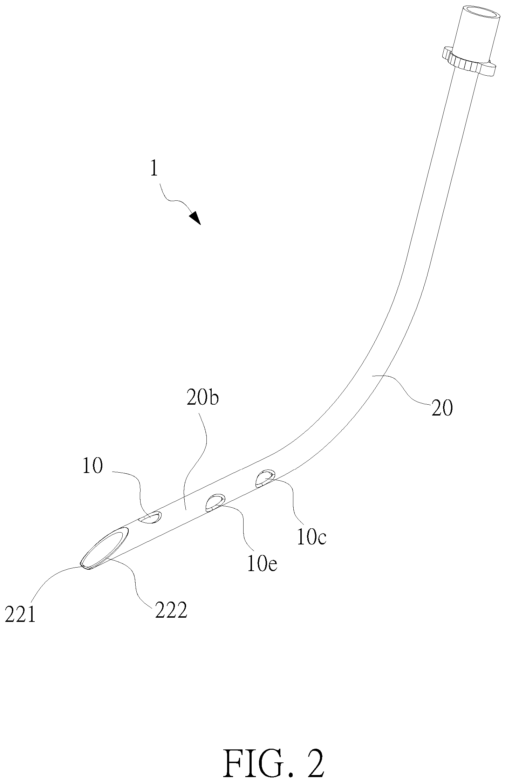

As shown in FIG. 1 and FIG. 2, in this embodiment, the nasopharyngeal airway 1 of the present invention includes a plurality of fenestrations 10, 10a, 10b and a curved tube 20. The curved tube 20 includes a curved front section 22. The curved front section 22 includes a front end 221 and an oblique surface 222. The fenestrations 10, 10a, 10b are provided in the curved front section 22. The curved tube 22 has a curved tube length D1 and the curved front section 22 has a front length D2, where

$$D2 \leq \frac{1}{3}D1$$

. According to one embodiment of the present invention, the curved tube length D1 can be 12 cm < D1 < 18 cm or 14 cm < D1 < 18 cm; the front length D2 can be 2 cm < D2 < 6 cm or 3 cm < D2 < 6 cm. However, the curved tube length D1 and the front length D2 are not limited to the abovementioned length ranges, for the curved tube length D1 and the front length D2 may vary

$$D2 \leq \frac{1}{3}D1$$

as long as the criterion of is satisfied. As shown in FIG. 1, the front length D2 is the distance between the front end 221 and the farthest opening 10b. In this embodiment, the length L of the oblique surface 222 is between 0.6 cm and 2.5 cm, or between 1 cm and 2.5 cm, and the opening 10 closest to the front end 221 is disposed at a distance of 1 cm to 1.5 cm from the front end 221. In other words, the nasopharyngeal airway 1 of the present invention provides an opening 10 at a position 1 cm to 1.5 cm from the front end 221. Please note the arrangements of the fenestrations are not limited to the above embodiment, for the opening 10 can also be provided directly at the front end 221, and the fenestrations can be provided within the front length D2.

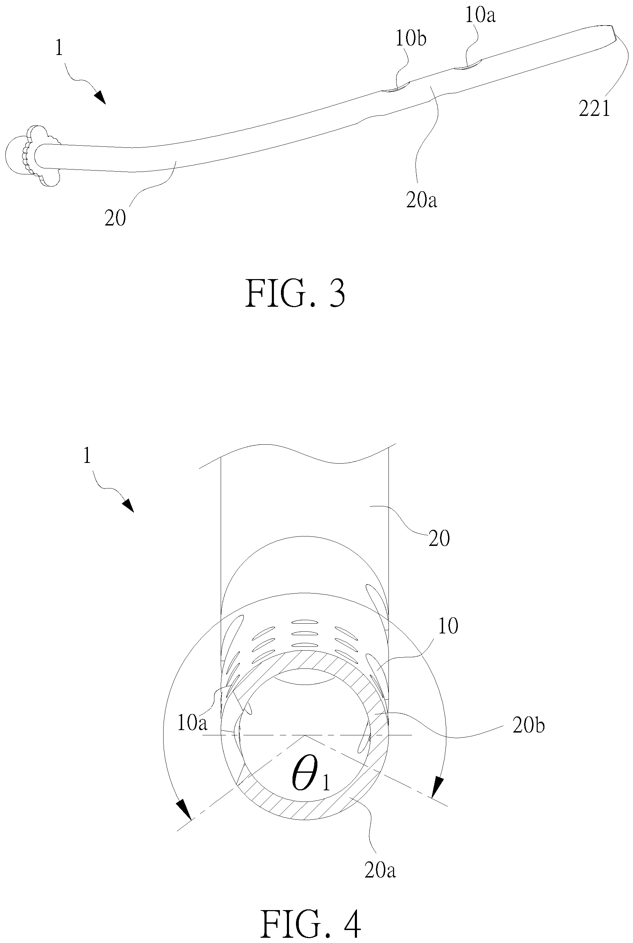

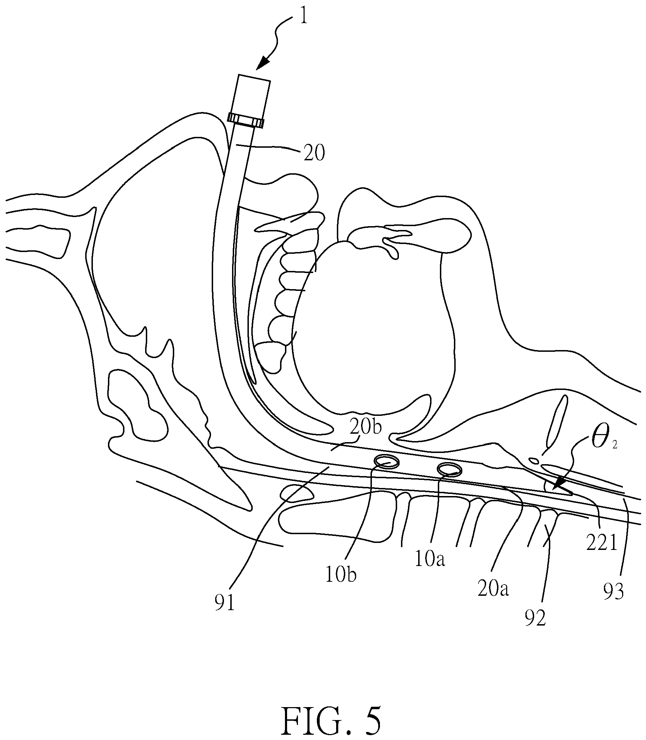

As shown in FIG. 3 and FIG. 4, the curved tube 20 has a curved inner side 20a and a curved outer side 20b. No fenestrations are provided in the curved inner side 20a. The fenestrations 10, 10a, 10b, 10c are provided discretely in the curved outer side 20b at distances of 1 cm to 1.5 cm from the front end 221. As shown in FIG. 5, after the nasopharyngeal airway 1 of the present invention is placed into a human pharynx 91 via the nasal cavity, the curved outer side 20b is the side of the nasopharyngeal airway 1 adjacent to the human pharynx 91, and the curved inner side 20a is the side of the nasopharyngeal airway 1 adjacent to the human cervical vertebra 92. When the nasopharyngeal airway 1 is placed into the human pharynx 91, in this embodiment, the length of the front end 221 of the nasopharyngeal airway 1 of the present invention can enter the human esophagus 93 such that the fenestrations 10a, 10b located in the curved outer side 20b can provide oxygen for the patient in the upper airway. In order to secure the ventilation volume of the nasopharyngeal airway 1 of the present invention, an open area of the plurality of fenestrations 10, 10a, 10b, 10c is equal to 3% to 40%, 10% to 40% or 10% to 30% of the area of the curved front section 22. Alternatively, it is also feasible to provide at least one and at most five fenestrations discretely within the unit area of one square centimeter of the curved front section 22. Please note that, according to one embodiment of the present invention, the curved inner side 20a and the curved outer side 20b can be observed in the cross-sectional drawing of the curved tube 20 as shown in FIG. 4. If the cross-sectional axle center is designated as the circle center, the curved inner side 20a covers the arc-shaped area within the central angle $\theta_1$, which is around 120 degrees, under the circle center, as shown in FIG. 4, and the curved outer side 20b covers all of the remaining area by excluding the area covered by the curved inner side 20a. In FIG. 5, the inclination angle $\theta_2$ of the oblique surface 222 is, but is not limited to, around 45 degrees. The inclination angle $\theta_2$ is between 80 degrees and 20 degrees.

Because of the provision of fenestrations in the curved front section 22 of the nasopharyngeal airway 1 of the present invention, as well as the insertion of the front end 221 into the human esophagus 93 when the nasopharyngeal airway 1 is placed into the human pharynx 91, even if the healthcare provider cannot precisely position the ventilation hole located in the front tip of the nasopharyngeal airway 1 of the present invention in the patient's upper airway, oxygen can still be provided to the patient via the opening 10 provided in the curved front section 22. As a result, the risk of oxygen deprivation due to inaccurate nasopharyngeal airway positioning is reduced, and the lack of precision in placement of the nasopharyngeal airway in an oxygen provision position is therefore resolved.

Although the present invention has been explained in relation to its preferred embodiments, it is to be understood that many other possible modifications and variations can be made without departing from the spirit and scope of the invention as hereinafter claimed.

## Claims

1. A nasopharyngeal airway (1), comprising:

   a plurality of fenestrations(10, 10a, 10b); and
   a curved tube(20), the curved tube(20) including an curved front section(22) and the curved front section(22) including a front end(221), wherein the plurality of fenestrations(10, 10a, 10b) are provided in the curved front section(22), the curved tube(20) has a curved tube length D1, and the curved front section(22) has an front

   $$D2 \leq \frac{1}{3}D1$$

   length D2, where .

2. The nasopharyngeal airway (1) as claimed in claim 1, wherein an open area of the plurality of fenestrations(10, 10a, 10b) covers 3% to 40% of the area of the curved front section(22).

3. The nasopharyngeal airway (1) as claimed in claim 2, wherein when the nasopharyngeal airway (1) is placed into a human pharynx(91), the front end(221) enters a human esophagus(93).

4. The nasopharyngeal airway (1) as claimed in claim 2, wherein the curved tube length D1 is 10 cm < D1 < 22 cm.

5. The nasopharyngeal airway (1) as claimed in claim 4, wherein when the nasopharyngeal airway (1) is placed into a human pharynx(91), the front end(221) enters a human esophagus(93).

6. The nasopharyngeal airway (1) as claimed in claim 4, wherein a plurality of fenestrations(10, 10a, 10b) are provided near the front end(221).

**7.** The nasopharyngeal airway (1) as claimed in claim 6, wherein when the nasopharyngeal airway (1) is placed into a human pharynx(91), the front end(221) enters a human esophagus(93).

**8.** The nasopharyngeal airway (1) as claimed in claim 6, wherein the curved tube(20) has a curved inner side(20a) and a curved outer side(20b), and the plurality of fenestrations(10, 10a, 10b) are provided in the curved outer side(20b).

**9.** The nasopharyngeal airway (1) as claimed in claim 8, wherein when the nasopharyngeal airway(1) is placed into a human pharynx(91), the front end enters(221) a human esophagus(93).

**10.** The nasopharyngeal airway (1) as claimed in claim 8, wherein each of the fenestrations(10, 10a, 10b) is provided discretely in the curved outer side(20b) at a distance of 1 cm to 1.5 cm from the front end(221).

**11.** The nasopharyngeal airway (1) as claimed in claim 10, wherein when the nasopharyngeal airway(1) is placed into a human pharynx(91), the front end(221) enters a human esophagus(93).

**12.** The nasopharyngeal airway (1) as claimed in claim 1, wherein within the unit area of one square centimeter of the curved front section(22), at least one and at most five fenestrations(10, 10a, 10b) are provided discretely therein.

**13.** The nasopharyngeal airway (1) as claimed in claim 1, wherein the curved front section(22) comprises an oblique surface(222), and an inclination angle $\theta_2$ is between 80 degrees and 20 degrees.

**14.** The nasopharyngeal airway (1) as claimed in claim 13, wherein the inclination angle $\theta_2$ is 45 degrees.

**15.** The nasopharyngeal airway (1) as claimed in claim 1, wherein when the nasopharyngeal airway (1) is placed into a human pharynx(91), the front end (221)enters a human esophagus(93).

FIG. 1

FIG. 2

FIG. 3

FIG. 4

FIG. 5

Europäisches
Patentamt

European
Patent Office

Office européen
des brevets

# EUROPEAN SEARCH REPORT

**Application Number**

EP 24 19 0001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 203 577 094 U (ZOU DEWEI) 7 May 2014 (2014-05-07) * figure 1 * | 1-15 | INV. A61M16/04 |
| A | US 11 623 058 B2 (WEDGE THERAPEUTICS LLC [US]) 11 April 2023 (2023-04-11) * figure 3c * | 1-15 | |
| A | CN 215 741 132 U (ANHUI SHUOYUAN MEDICAL DEVICES CO LTD) 8 February 2022 (2022-02-08) * figure 1 * | 1-15 | |

**TECHNICAL FIELDS SEARCHED (IPC)**

A61M

The present search report has been drawn up for all claims

| Place of search | Date of completion of the search | Examiner |
|---|---|---|
| Munich | 22 November 2024 | Louarn, Arzhur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 0001

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2024

| Patent document cited in search report | | Publication date | Patent family member(s) | | Publication date |
|---|---|---|---|---|---|
| CN 203577094 | U | 07-05-2014 | NONE | | |
| US 11623058 | B2 | 11-04-2023 | US | 2021093818 A1 | 01-04-2021 |
| | | | WO | 2018200063 A1 | 01-11-2018 |
| CN 215741132 | U | 08-02-2022 | NONE | | |

EPO FORM P0459

For more details about this annex : see Official Journal of the European Patent Office, No. 12/82